# EUROPEAN PATENT APPLICATION

(11) **EP 4 725 407 A1**
(43) Date of publication of application: **15.04.2026**
(21) Application number: 25204958.0
(22) Date of filing: 26.09.2025
(51) Int. Cl.: A61B 5/024, A61B 5/00, A61B 5/1455

(54) **PHYSIOLOGICAL SIGNAL SENSING METHOD AND APPARATUS, AND ELECTRONIC DEVICE**

(30) Priority: 11.10.2024 CN 202411419783
(71) Applicant: Shenzhen Goodix Technology Co., Ltd., Shenzhen, Guangdong 518045 (CN)
(72) Inventor: CHENG, Shuqing, Shenzhen, 518045 (CN)
(74) Representative: Metida

(57) **Abstract**

The example of the present application provides a physiological signal sensing method and apparatus, and an electronic device, where an apparatus part includes: one or more light emitters which are used for emitting first light; one or more photoelectric sensors which are used for detecting second light obtained through reflecting the first light via biological tissue and generating a corresponding first signal; a capacitive sensor which is used for detecting changes of a capacitor which is in contact with the biological tissue and generating a corresponding second signal; and a signal processing module which is used for carrying out motion artifact elimination on the first signal according to the second signal, so as to obtain a third signal. In some examples, the one or more photoelectric sensors are multiplexed as the capacitive sensor. Spatial correlation between the first signal and the second signal is higher, and then a better motion artifact elimination effect is achieved.

## Description

### TECHNICAL FIELD

The present application relates to the technical field of physiological signal sensing, and specifically relates to a physiological signal sensing method and apparatus, and an electronic device.

### BACKGROUND

At present, in wearable electronic devices such as smartwatches, bracelets, rings, head-mounted devices, and headphones, monitoring for physiological parameters such as a heart rate, a blood pressure, and blood oxygen saturation is realized by means of a photoplethysmography (abbreviated as PPG) technology. However, a big challenge has always been that users have different wearing habits of elasticity, so that different degrees of motion interference (commonly known as motion artifacts) of PPG are caused, for example, when the users are in a sports scene such as running or rope skipping, arms and wrists may swing to different degrees, and in the case of loose wearing, strong motion artifacts may cause heart rate signals to be submerged in noise, so that accurate measurement cannot be realized, and sometimes even heart rates cannot be monitored.

A present method in the industry is carrying out motion artifact elimination by means of motion sensors (such as acceleration sensors and inertial sensors), but is limited in filtering effect.

### SUMMARY

In view of the above problems, the example of the present application provides a physiological signal sensing method and apparatus, and an electronic device, for solving the above technical problems.

In a first aspect, the example of the present application provides a physiological signal sensing apparatus, and the physiological signal sensing apparatus includes: one or more light emitters which are used for emitting first light; one or more photoelectric sensors which are used for detecting second light obtained through reflecting the first light via biological tissue and generating a corresponding first signal; a capacitive sensor which is used for detecting changes of a capacitor which is in contact with the biological tissue and generating a corresponding second signal; and a signal processing module which is used for carrying out motion artifact elimination on the first signal according to the second signal, so as to obtain a third signal.

**In** some possible examples, the one or more photoelectric sensors are multiplexed as the capacitive sensor.

**In** some possible examples, the second signal is generated through detecting self-capacitance of at least one of the photoelectric sensors, and/or mutual capacitance of at least one group of the photoelectric sensors, and each group of the photoelectric sensors includes two photoelectric sensors.

**In** some possible examples, the physiological signal sensing apparatus further includes an analog front-end, where the analog front-end includes: a switch module which is used for controlling a working mode of the photoelectric sensors, so as to enable the photoelectric sensors to generate the first signal or the second signal; a driving module which is used for driving the photoelectric sensors to generate the second signal; and an analog signal processing module which is used for processing the first signal and the second signal.

**In** some possible examples, the one or more light emitters are configured to emit the first light in a first period, and the one or more photoelectric sensors are configured to generate the first signal in the first period; and the capacitive sensor is configured to generate the second signal in a second period, where the second period is different from the first period.

**In** some possible examples, the signal processing module includes: a self-adaptive filtering unit which is used for carrying out weighting processing on the second signal to output a normalized fourth signal, and adjusting weighting parameters according to the third signal; and an elimination unit which is used for carrying out subtraction operation on the first signal and the second signal, so as to output the third signal.

**In** some possible examples, the signal processing module is configured to use a neural network model to process the first signal and the second signal, so as to output the third signal through the neural network model, where the third signal is a physiological signal.

**In** a second aspect, the example of the present application provides a physiological signal sensing method, and the physiological signal sensing method includes: emitting first light at biological tissue; detecting second light obtained through reflecting the first light via the biological tissue and generating a corresponding first signal; detecting changes of a capacitor which is in contact with the biological tissue and generating a corresponding second signal; and carrying out motion artifact elimination on the first signal according to the second signal, so as to obtain a third signal.

In some possible examples, the first light is emitted, the second light is detected, and the first signal is generated in the first period, and the changes of the capacitor are detected, and the second signal is generated in the second period, where the second period is different from the first period.

In some possible examples, the second light is detected and the first signal is generated through one or more photoelectric sensors in the first period, and the one or more photoelectric sensors are used as electrodes for detecting the changes of the capacitor and generating the second signal in the second period.

In some possible examples, the carrying out motion artifact elimination on the first signal according to the second signal, so as to obtain a third signal includes: carrying out weighting processing on the second signal to output a normalized fourth signal, and adjusting weighting parameters according to the third signal; and carrying out subtraction operation on the first signal and the second signal, so as to output the third signal.

In some possible examples, the carrying out motion artifact elimination on the first signal according to the second signal, so as to obtain a third signal includes: using a neural network model to process the first signal and the second signal, so as to output the third signal through the neural network model, where the third signal is a physiological signal.

In a third aspect, the example of the present application provides an electronic device, and the electronic device includes the above physiological signal sensing apparatus.

In a fourth aspect, the example of the present application provides an electronic device, and the electronic device includes: one or more light emitters which are used for emitting first light; one or more photoelectric sensors which are used for detecting second light obtained through reflecting the first light via biological tissue and generating a corresponding first signal; a capacitive sensor which is used for detecting changes of a capacitor which is in contact with the biological tissue and generating a corresponding second signal; a processor; and a memory storing a program, where the program includes instructions, and the processor is enabled to execute the above method when the instructions are executed by the processor.

In some possible examples, the one or more photoelectric sensors are multiplexed as the capacitive sensor.

In the related art, a motion sensor carries out a solution of motion artifact elimination, the motion sensor is located in an electronic device, while a PPG sensor is in contact with biological tissue, and spatial correlation between the motion sensor and the PPG sensor is not high, so that a low elimination effect is caused. According to the physiological signal sensing method and apparatus, and the electronic device, which are provided by the example of the present application, the photoelectric sensors detect the second light obtained through reflecting the first light which is emitted by the light emitters via the biological tissue and generate the corresponding first signal, the capacitive sensor detects the changes of the capacitor which is in contact with the biological tissue and generates the corresponding second signal, both the photoelectric sensors and the capacitive sensor are in contact with the biological tissue, so that spatial correlation between the first signal and the second signal which are generated by the photoelectric sensors and the capacitive sensor is higher, and then a better motion artifact elimination effect is achieved.

These aspects or other aspects of the present application will be clearer and easier to understand in the descriptions for the following examples.

### BRIEF DESCRIPTION OF DRAWINGS

In order to describe the technical solutions in the examples of the present application more clearly, a brief introduction for the drawings required in the descriptions for the examples will be provided below, apparently, the drawings in the descriptions below show merely some examples of the present application, and those skilled in the art may also derive other drawings from these drawings without making creative efforts.
FIG. 1 is a schematic diagram of a system architecture of a physiological signal sensing apparatus which is provided by the example of the present application;
FIG. 2 is a schematic diagram of a system architecture of a physiological signal sensing apparatus multiplexing photoelectric sensors, which is provided by the example of the present application;
FIG. 3A is a schematic structure diagram of a self-capacitance detection manner;
FIG. 3B is a schematic structure diagram of a mutual capacitance detection manner;
FIG. 4 is a schematic diagram of an exemplary light path structure;
FIG. 5A is a schematic diagram of a motion artifact elimination manner;
FIG. 5B is a schematic diagram of another motion artifact elimination manner;
FIG. 6 is a schematic diagram of a system architecture of an electronic device which is provided by the example of the present application; and
FIG. 7 is a flow diagram of a physiological signal sensing method.

### DETAILED DESCRIPTION

The implementation manners of the present application are described below in detail, and instances of the implementation manners are shown in the drawings, where the same or similar mark numbers from beginning to end represent the same or similar components or components with the same or similar functions. The implementation manners described below with reference to the drawings are exemplary, and are merely used for explaining the present application, rather than being understood as limiting the present application.

In order to enable those skilled in the art to better understand the solutions of the present application, the technical solutions in the examples of the present application will be clearly and completely described below in conjunction with the drawings in the examples of the present application. Apparently, the described examples are merely part rather than all of the examples of the present application. On the basis of the examples in the present application, all other examples obtained by those skilled in the art without creative efforts shall fall within the protection scope of the present application.

In the examples of the present application, it needs to be noted that, the relationship terms herein, such as the first and the second, are merely used for distinguishing one entity or operation from another entity or operation, and do not necessarily require or imply that any actual relationship or sequence exists between these entities or operations.

Moreover, the terms 'including', 'containing', or any other variations thereof are intended to encompass non-exclusive inclusions, so that a process, method, article, or device that includes a series of elements not only includes those elements, but also includes other elements not listed explicitly, or further includes elements inherent to such process, method, article, or device. In the absence of more limitations, the element defined by the statement 'including one...' does not exclude the existence of other identical elements in the process, method, article, or device that includes the element.

In the descriptions for the examples of the present application, the words such as 'example' or 'for example' are used for indicating examples, explanations, or descriptions. Any example or design solution described as 'example' or 'for example' in the examples of the present application shall not be interpreted as being more preferred or having more advantages than another example or design solution. The use for the words such as 'example' or 'for example' is intended to present relative concepts in a clear manner.

In addition, the term 'a plurality of in the examples of the present application refers to two or more, and in view of this, the term 'a plurality of may also be understood as 'at least two' in the examples of the present application. The term 'at least one' may be understood as one or more, such as one, two, or more. For example, including at least one refers to including one, two, or more, without limiting which ones are included, for example, including at least one of A, B, and C may be including A, B, C, A and B, A and C, B and C, or A and B and C.

It needs to be noted that, in the examples of the present application, the term 'and/or' describes an association relationship of associated objects, indicating that there may be three relationships, for example, A and/or B may be represented as: A exists alone, A and B exist at the same time, or B exists alone. In addition, the character '/', unless otherwise specified, generally represents that the associated objects before and after are in a 'or' relationship.

It needs to be pointed out that, the 'connection' in the examples of the present application may be understood as electric connection, and connection between two electric components may be direct connection or indirect connection between the two electric components. For example, connection between A and B may be that A and B are directly connected or indirectly connected through one or more other electric components.

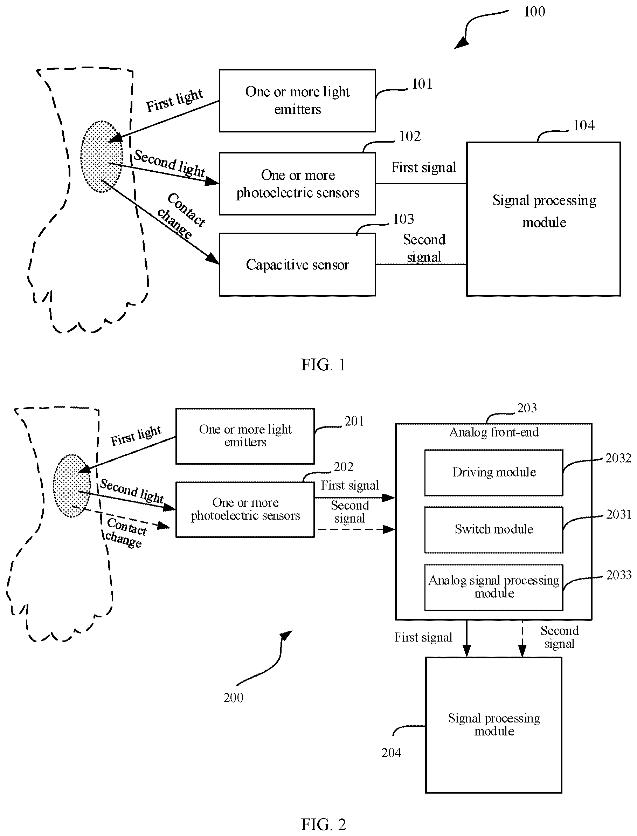

FIG. 1 is a schematic structure diagram of a physiological signal sensing apparatus which is provided by the example of the present application, and as shown in FIG. 1, the physiological signal sensing apparatus 100 may include: one or more light emitters 101, one or more photoelectric sensors 102, a capacitive sensor 103, and a signal processing module 104. The one or more light emitters 101 may be configured to emit first light, the one or more photoelectric sensors 102 may be configured to detect second light obtained through reflecting the first light via biological tissue (such as skin) and generate a corresponding first signal, where the first signal includes a physiological signal and a motion artifact noise, and the motion artifact noise is at least partially caused by changes of contact between the one or more photoelectric sensors 102 and the biological tissue. The capacitive sensor 103 is used for detecting changes of a capacitor which is in contact with the biological tissue and generating a corresponding second signal, in a motion process, the contact between the biological tissue and the capacitive sensor 103 may change, such as contact changes of a position, a distance, etc., which will cause the changes of the capacitor, and the second signal can indicate the contact changes caused by motion, and includes the motion artifact noise. The signal processing module 104 may carry out motion artifact elimination on the first signal according to the second signal, so as to obtain a third signal.

In some specific examples, the capacitive sensor 103 generates the second signal through detecting self-capacitance of at least one electrode, and the detection may be realized through one electrode in the example. In some possible examples, the capacitive sensor 103 generates the second signal through detecting mutual capacitance of at least one group of electrodes, each group of electrodes includes two electrodes, and position changes of the plurality of electrodes relative to the biological tissue can be better embodied in the example. In some possible examples, the capacitive sensor 103 generates the second signal through detecting the above self-capacitance and mutual capacitance, such as detecting the self-capacitance of the first electrode to generate a first sub-signal, detecting the mutual capacitance of the second electrode and the third electrode to generate a second sub-signal, where the second signal may include the first sub-signal and the second sub-signal.

In some specific examples, the one or more light emitters 101 may be configured to emit the first light in a first period, and the one or more photoelectric sensors 102 are configured to generate the first signal in the first period, where the first signal includes a physiological signal and a motion artifact noise. The capacitive sensor 103 may be configured to generate the second signal in a second period which is different from the first period, where the second signal includes a motion artifact noise.

In some possible examples, the one or more photoelectric sensors are multiplexed as the capacitive sensor, and as shown in FIG. 2, the physiological signal sensing apparatus 200 may include: one or more light emitters 201, one or more photoelectric sensors 202, and a signal processing module 204. The one or more light emitters 201 may be configured to emit first light, the one or more photoelectric sensors 202 may be configured to detect second light obtained through reflecting the first light via biological tissue (such as skin) and generate a corresponding first signal, where the first signal includes a physiological signal and a motion artifact noise. The one or more photoelectric sensors 202 may also be multiplexed to detect changes of a capacitor which is in contact with the biological tissue and generate a corresponding second signal. Capacitance detection is carried out through multiplexing the photoelectric sensors, so that the photoelectric sensors are enabled to sense light information and detect electric information on an original basis, and then while respective advantages of the capacitance detection and optical detection are combined, design complexity of a circuit does not need to be increased.

Continuing to refer to FIG. 2, in a specific realization, the physiological signal sensing apparatus 200 further includes an analog front-end 203. The analog front-end 203 may include a switch module 2031, a driving module 2032 and an analog signal processing module 2033. The switch module 2031 is used for controlling a working mode of the photoelectric sensors 202, so as to enable the photoelectric sensors 202 to generate the first signal or the second signal, and specifically, the working mode may include a photoelectric detection mode and a contact detection mode, in the photoelectric detection mode, second light obtained through reflecting the first light via biological tissue (such as skin) is detected and a corresponding first signal is generated; and in the contact detection mode, changes of a capacitor which is in contact with the biological tissue are detected and a corresponding second signal is generated. The driving module 2032 may drive the photoelectric sensors 202 to generate the second signal. The analog signal processing module 2033 may process the first signal and the second signal, where the processing includes signal amplification, analog-to-digital conversion, filtering, etc.

In some specific examples, the second signal is generated through detecting self-capacitance of at least one of the photoelectric sensors 202, detection for contact changes may be realized through one photoelectric sensor 202 in the example, and the method can be applied to scenes with only one photoelectric sensor 202, and scenes that include a plurality of the photoelectric sensors 202.

In some possible examples, the second signal is generated through detecting mutual capacitance of at least one group of the photoelectric sensors 202, each group of the photoelectric sensors 202 includes two photoelectric sensors 202, and position changes of the plurality of photoelectric sensors 202 relative to the biological tissue can be better embodied in the example.

In some possible examples, the one or more light emitters 201 are configured to emit the first light in a first period, and the one or more photoelectric sensors 202 are configured to generate the first signal in the first period. The the one or more photoelectric sensors 202 are configured to generate the second signal in a second period, where the second period is different from the first period. Please refer to FIG. 2, in the first period, the one or more light emitters 201 are controlled to emit first light, and the switch module 2031 is controlled to enable the photoelectric sensors 202 to work in the photoelectric detection mode, and in the photoelectric detection mode, second light obtained through reflecting the first light via biological tissue (such as skin) is detected and a corresponding first signal is generated; and in the second period, the one or more light emitters 201 are controlled to stop emitting the first light, and the switch module 2031 is controlled to enable the photoelectric sensors 202 to work in the contact detection mode, and in the contact detection mode, the one or more photoelectric sensors 202 detect changes of a capacitor which is in contact with the biological tissue and generate a corresponding second signal. Specifically, in a realization, a specific photoelectric sensor 202 may be controlled to work in the contact detection mode, and the photoelectric sensor 202 is used as an electrode, and self-capacitance of the electrode is detected to generate the second signal. In another realization, two specific photoelectric sensors 202 may be controlled to work in the contact detection mode, the two photoelectric sensors 202 are used as electrodes, and mutual capacitance of the two electrodes is detected to generate the second signal.

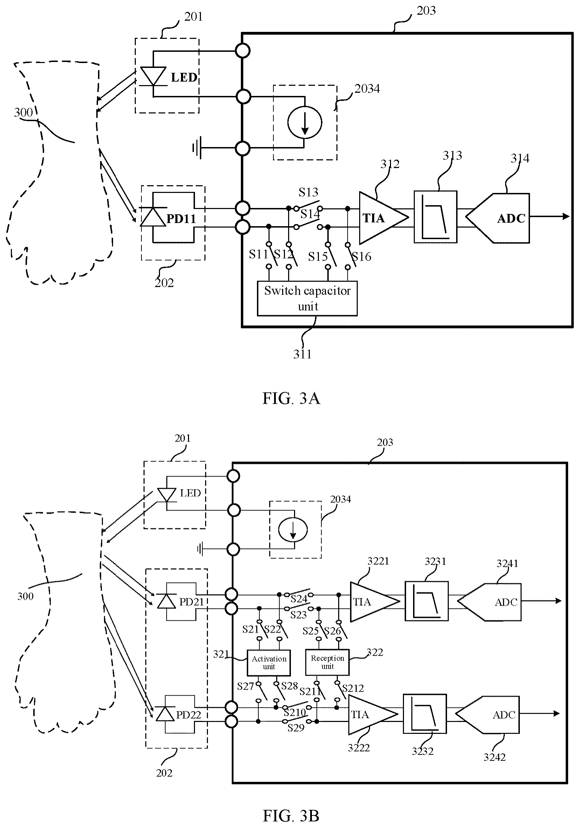

FIG. 3A and FIG. 3B respectively are schematic circuits diagrams of detecting the self-capacitance and the mutual capacitance through multiplexing the photoelectric sensors, and referring to FIG. 3A and FIG. 3B, the physiological signal sensing apparatus 200 may be worn on a wrist 300 of a user. The light emitters 201 may be configured to emit light to the wrist 300, the photoelectric sensors 202 may be configured to detect light obtained through reflecting the light via the wrist 300 and generate a corresponding first signal, and the photoelectric sensors 202 may further be configured to detect changes of a capacitor which is in contact with the wrist 300 and generate a corresponding second signal. Please refer to FIG. 3A and FIG. 3B, the light emitter 201 may be a light-emitting diode (abbreviated as LED), and the light-emitting diode may include a single-color light-emitting diode or a multi-color light-emitting diode, and the photoelectric sensor 202 may be a photodiode (abbreviated as PD). In the example of the present application, by means of conduction characteristics of a photosensitive part of the photodiode, the photodiodes are multiplexed to sense changes of a capacitor which is in contact with a human body. Continuing to refer to FIG. 3A and FIG. 3B, in a specific realization, the analog front-end 203 may further include a light emitter driving module 2034. The light emitter driving module 2034 may drive the light emitters 201 to emit light.

Please continue to refer to FIG. 3A, in a typical realization of detecting the self-capacitance, the switch module 2031 may include a first switch S11, a second switch S12, a third switch S13, a fourth switch S14, a fifth switch S15, and a sixth switch S16; the driving module 2032 may include a switch capacitor unit 311; and the analog signal processing module 2033 may include a trans-impedance amplifier 312, a filter 313, and an analog-to-digital converter (ADC) 314. In FIG. 3A, the photodiode PD 11 is multiplexed as the capacitive sensor 202, and it should be understood that, the physiological signal sensing apparatus 200 may further include another one or more photodiodes, and for simplicity, only the photodiode PD 11 are shown in FIG. 3A. A typical detection process may include: in the first period, the one or more light-emitting diodes emit light to the wrist 300, the third switch S13 and the fourth switch S14 are turned on, the first switch S11, the second switch S12, the fifth switch S15, and the sixth switch S16 are turned off, the photodiode PD 11 detect light obtained through reflecting the light via the wrist 300 and generate a first signal, and the analog signal processing module 2033 carries out amplification, filtering, and analog-digital conversion on the first signal to obtain the first signal in a digital form; and in the second period which is different from the first period, the light emitters 201 stop emitting light to the wrist 300, the third switch S13 and the fourth switch S14 are turned off, the first switch S11, the second switch S12, the fifth switch S15, and the sixth switch S16 are turned on, the switch capacitor unit 311 drives the photodiode PD 11 to detect changes of a capacitor which is in contact with the wrist 300 and generate a corresponding second signal, and the analog signal processing module 2033 carries out amplification, filtering, and analog-digital conversion on the second signal to obtain the second signal in a digital form.

Please continue to refer to FIG. 3B, in a typical realization of detecting the mutual capacitance, the switch module 2031 may include a first switch S21, a second switch S22, a third switch S23, a fourth switch S24, a fifth switch S25, a sixth switch S26, a seventh switch S27, an eighth switch S28, a ninth switch S29, a tenth switch S210, an eleventh switch S211, and a twelfth switch S212; the driving module 2032 may include an activation unit 321 and a reception unit 322; the analog signal processing module 2033 may include a trans-impedance amplifier 3221, a filter 3231, and an analog-to-digital converter (ADC) 3241 which correspond to the photodiode PD 21, as well as a trans-impedance amplifier 3222, a filter 3232, and an analog-to-digital converter (ADC) 3242 which correspond to the photodiode PD 22. In FIG. 3B, the photodiode PD 21 and the photodiode PD 22 are multiplexed as the capacitive sensor 202, and it should be understood that, the physiological signal sensing apparatus 200 may further include another one or more photodiodes, and for simplicity, only the photodiode PD 21 and the photodiode PD 22 are shown in FIG. 3B. A typical detection process may include: in the first period, the one or more light-emitting diodes emit light to the wrist 300, the third switch S23, the fourth switch S24, the ninth switch S29, and the tenth switch S210 are turned on, the first switch S21, the second switch S22, the fifth switch S25, the sixth switch S26, the seventh switch S27, the eighth switch S28, the eleventh switch S211, and the twelfth switch S212 are turned off, the photodiode PD 21 and the photodiode PD 22 detect light obtained through reflecting the light via the wrist 300 and respectively generate the first signal, and the analog signal processing module 2033 carries out amplification, filtering, and analog-digital conversion on each first signal to obtain the first signal in a digital form; and in the second period which is different from the first period, the light emitters 201 stop emitting light to the wrist 300, the third switch S23, the fourth switch S24, the seventh switch S27, the eighth switch S28, the ninth switch S29, and the tenth switch S210 are turned off, the first switch S21 and the second switch S22 are turned on to excite the photodiode PD 21, then the ninth switch S29, the tenth switch S210, the eleventh switch S211, and the twelfth switch S212 are turned on to receive, the second signal is generated by the photodiode PD 22, and the analog signal processing module 2033 carries out amplification, filtering, and analog-digital conversion on the second signal to obtain the second signal in a digital form. In addition, in the second period, the photodiode PD 22 may be excited, and then the second signal is generated by the photodiode PD 21.

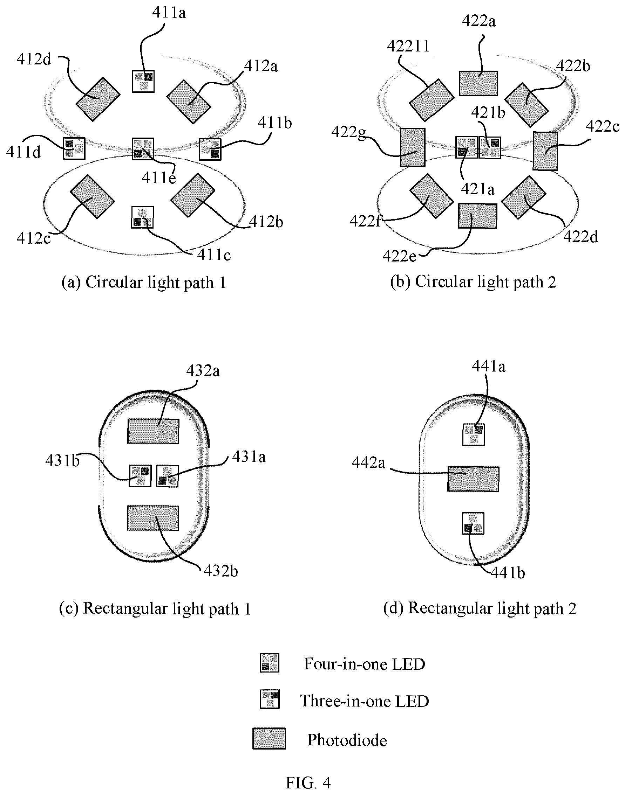

Please refer to FIG. 4 which shows a typical light path structure, where a part (a) shows a circular light path 1. In the circular light path 1, light emission sensors and the photoelectric sensors are distributed in a circular ring at intervals, the interior of the circular ring may include one or more light emitters, specifically, photodiodes 412a, 412b, 412c and 412d, as well as light-emitting diodes 411a, 411b, 411c and 411d, are arranged on the circular ring, a light-emitting diode 411e is arranged in the circular ring, and moreover, in a specific realization, a number of the photodiodes and a number of the light-emitting diodes may be set according to product needs. In the example of the present application, if the detection for the contact changes is realized in a self-capacitance detection manner, any one of the photodiodes 412a, 412b, 412c and 412d may be multiplexed as the capacitive sensor; if the detection for the contact changes is realized in a mutual capacitance detection manner, any two of the photodiodes 412a, 412b, 412c and 412d may be multiplexed as the capacitive sensor, and in some realizations, two adjacent photodiodes are used as two electrodes of the capacitive sensor, such as photodiodes 412a and 412b; and in some realizations, two photodiodes on a diagonal line are used as two electrodes of the capacitive sensor, such as photodiodes 412a and 412c.

Please continue to refer to FIG. 4, a part (b) shows a circular light path 2. In the circular light path 2, the photoelectric sensors are distributed in a circular ring, and one or more light emitters are arranged in the circular ring. Specifically, photodiodes 422a, 422b, 422c, 422d, 422e, 422f, 422g and 422h are arranged on the circular ring, and photodiodes 421a and 421b are arranged in the circular ring, and moreover, in a specific realization, a number of the photodiodes and a number of the light-emitting diodes may be set according to product needs. Similarly, if the detection for the contact changes is realized in a self-capacitance detection manner, any one of the photodiodes 422a, 422b, 422c, 422d, 422e, 422f, 422g and 422h may be multiplexed as the capacitive sensor; if the detection for the contact changes is realized in a mutual capacitance detection manner, any two photodiodes may be multiplexed as the capacitive sensor, and in some realizations, two adjacent photodiodes are used as two electrodes of the capacitive sensor, such as photodiodes 422a and 422b; and in some realizations, two photodiodes on a diagonal line are used as two electrodes of the capacitive sensor, such as photodiodes 412a and 412e, and a distance between the two photodiodes on the diagonal line is larger, so that the contact changes in a larger range can be detected.

Please continue to refer to FIG. 4, a part (c) shows a rectangular light path 1. Specifically, in the rectangular light path 1, the photodiodes and the light-emitting diodes are in a rectangular shape as a whole, the photodiodes 432a and 432b are respectively arranged at the both sides of a rectangle, and the light-emitting diodes 431a and 431b are arranged at the middle. If the detection for the contact changes is realized in a self-capacitance detection manner, the photodiode 432a or 432b may be multiplexed as the capacitive sensor; and if the detection for the contact changes is realized in a mutual capacitance detection manner, the photodiodes 432a and 432b are used as two electrodes of the capacitive sensor.

Please continue to refer to FIG. 4, a part (d) shows a rectangular light path 2. Specifically, in the rectangular light path 2, the photodiodes and the light-emitting diodes are in a rectangular shape as a whole, the the light-emitting diodes 441a and 441b are respectively arranged at the both sides of a rectangle, and the photodiode 442a is arranged at the middle. In the light path structure, there is only one photodiode, that is, the photodiode 442a, the photodiode 442a is multiplexed as the capacitive sensor, and the contact changes are detected in a self-capacitance detection manner.

As shown in FIG. 4, the contact changes may be detected in the self-capacitance detection manner in all the light path structures of (a), (b), (c), and (d). The contact changes may be detected in the mutual capacitance detection manner in all the light path structures of (a), (b) and (c), while the contact changes can be only detected in the self-capacitance detection manner in the light path structure of (d). It may be seen that, the two solutions have respective advantages and disadvantages, the self-capacitance detection solution can support a wider range of light path designs, while the mutual capacitance detection solution can better embody position changes of the plurality of photoelectric sensors relative to the skin of a human body. The self-capacitance detection solution and the mutual capacitance detection solution may also be combined to realize a self-capacitance-and-mutual capacitance integrated detection solution.

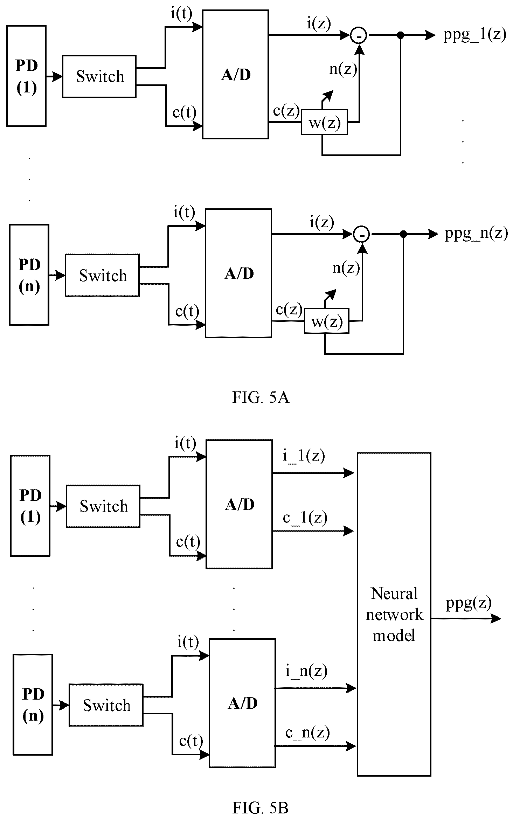

In some possible implementation manners, the signal processing module 204 may carry out motion artifact elimination on the first signal to obtain a third signal after motion artifact elimination, and a physiological signal may be generated on the basis of the third signal. In the implementation manner, a physiological signal generation process is separated from a motion artifact elimination process, and motion artifact processing does not influence a physiological signal processing algorithm. As shown in FIG. 5A, the signal processing module 204 may include: a self-adaptive filtering unit and an elimination unit. The self-adaptive filtering unit is used for carrying out weighting processing on the second signal to output a normalized fourth signal, and adjusting weighting parameters according to the third signal. The elimination unit is used for carrying out subtraction operation on the first signal and the second signal, so as to output the third signal. The signal processing module 104 is similar to the elimination unit, which will not be elaborated herein.

Continuing to refer to FIG. 5A, the first signal is a current signal i(t), the second signal is a capacitance signal c(t), the current signal i(t) and the capacitance signal c(t) are subjected to analog-to-digital conversion (A/D) to obtain i(z) and c(z) respectively, it may be understood that the i(z) contains a motion artifact and a needed PPG signal, while the c(z) only contains a motion artifact signal, however, due to different units of the two signals, normalization processing is needed for noise reduction. Therefore, the self-adaptive filtering unit carries out weighting processing on the signal c(z) through a weighting function w(z) and realizes normalization with the signal i(z), and converts the signal c(z) and the signal i(z) into a final motion artifact noise signal n(z) capable of being directly subtracted, the elimination unit (shown as '-' in the drawing) carries out subtraction operation, that is, i(z) - n(z), so as to obtain the needed PPG signal ppg(z), and meanwhile, due to different configurations, relative values between a light current and the capacitance signal may change. Therefore, weighting parameters of the self-adaptive filtering unit may be dynamically adjusted according to the finally-output PPG signal, so as to achieve the best noise reduction effect. The extracted PPG signal may be adapted to an upper-layer application algorithm.

In some possible examples, referring to FIG. 5B, the signal processing module 204 may be used for using a neural network model to process the first signal and the second signal, so as to output the third signal through the neural network model, where the third signal is a physiological signal. The signal processing module 104 is similar to the elimination unit, which will not be elaborated herein.

Continuing to refer to FIG. 5B, the first signal is a current signal i(t), the second signal is a capacitance signal c(t), the current signal i(t) and the capacitance signal c(t) are subjected to analog-to-digital conversion (A/D) to obtain i(z) and c(z) respectively, and it may be understood that the i(z) contains a motion artifact and a needed PPG signal, while the c(z) only contains a motion artifact signal. The the capacitance signal c(z) is directly used as a source signal and input into the neural network model with the i(z), and a physiological signal is output through the neural network model, and the method is simple, and does not need design for a complex weighting function.

The example of the present application further provides an electronic device, and the electronic device may be the above physiological signal sensing apparatus 100 and physiological signal sensing apparatus 200, or include the above physiological signal sensing apparatus 100 and physiological signal sensing apparatus 200.

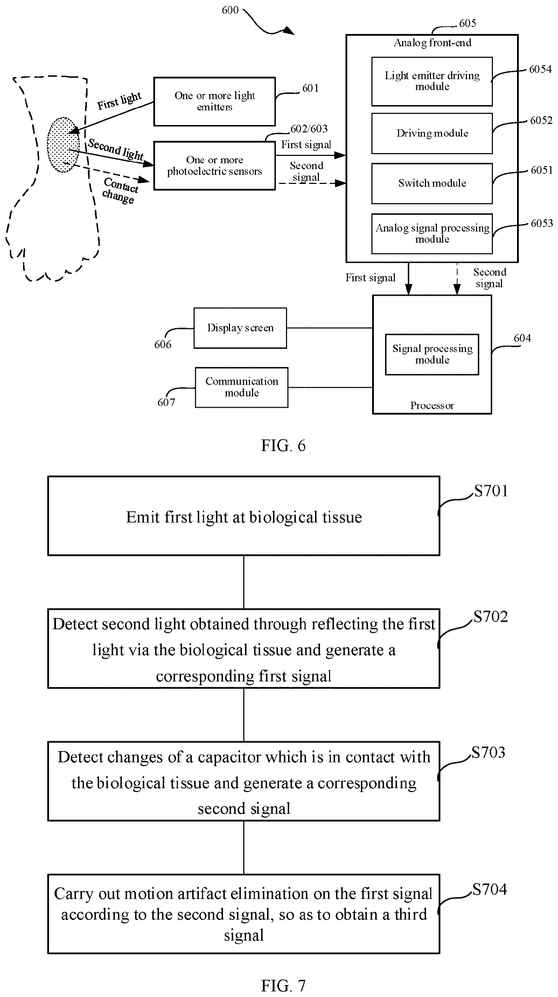

FIG. 6 is a schematic architecture diagram of a typical electronic device 600, and as shown in FIG. 6, the electronic device 600 may include: one or more light emitters 601, one or more photoelectric sensors 602, a capacitive sensor 603, and a processor 604. For simplicity, the example of the present application is specifically described below in an implementation manner of multiplexing the one or more photoelectric sensors 602 as the capacitive sensor 603.

The one or more light emitters 601 may be configured to emit first light, the one or more photoelectric sensors 602 may be configured to detect second light obtained through reflecting the first light via biological tissue (such as skin) and generate a corresponding first signal, where the first signal includes a physiological signal and a motion artifact noise, and the motion artifact noise is at least partially caused by changes of contact between the one or more photoelectric sensors 602 and the biological tissue. The photoelectric sensors 602 are multiplexed as the capacitive sensor 603 to detect changes of a capacitor which is in contact with the biological tissue and generate a corresponding second signal, in a motion process, the contact between the biological tissue and the capacitive sensor 603 may change, such as contact changes of a position, a distance, etc., which will cause changes of a capacitor, and the second signal can indicate the contact changes caused by motion, and includes the motion artifact noise. The processor 604 may be a central processing unit (CPU), a microcontroller (MCU), and other processors, and may include a signal processing module, and the signal processing module may carry out motion artifact elimination on the first signal according to the second signal, so as to obtain a third signal.

**In** a specific example, FIG. 4 may be referred to for light path structures of the one or more light emitters 601 and the one or more photoelectric sensors 602, and numbers and a layout relationship of the light emitters 601 and the photoelectric sensors 602 may be adjusted according to product needs.

Please continue to refer to FIG. 6, in a specific example, the electronic device 600 may further include a analog front-end 605, and the analog front-end 605 may include a switch module 6051, a driving module 6052, and an analog signal processing module 6053. The switch module 6051 is used for controlling a working mode of the photoelectric sensors 602, so as to enable the photoelectric sensors 602 to generate the first signal or the second signal, and specifically, the working mode may include a photoelectric detection mode and a contact detection mode, in the photoelectric detection mode, second light obtained through reflecting the first light via biological tissue (such as skin) is detected and a corresponding first signal is generated; and in the contact detection mode, changes of a capacitor which is in contact with the biological tissue are detected and a corresponding second signal is generated. The driving module 6052 may drive the photoelectric sensors 602 to generate the second signal. The analog signal processing module 6053 may process the first signal and the second signal, where the processing includes signal amplification, analog-to-digital conversion, filtering, etc. FIG. 3A or FIG. 3B may be referred to for a detailed structure of the analog front-end 605, which will not be elaborated herein.

Continuing to refer to FIG. 6, in a specific realization, the analog front-end 605 may further include a light emitter driving module 6054. The light emitter driving module 6054 may drive the light emitters 601 to emit light.

Please continue to refer to FIG. 6, in some specific examples, the electronic device 600 may further include a display screen 606, and the display screen 606 may be used for displaying images and providing a human-computer interaction interface for a user, such as displaying physiological signals such as a heart rate and blood oxygen, which are determined on the basis of the first signal and the second signal. The display screen 606 may be a touch-sensitive display for the user to carry out touch-control input, and the touch-control input may select to detect one or more physiological signals. Specifically, the user may start heart rate monitoring, blood oxygen monitoring, etc. through the touch-control input.

Please continue to refer to FIG. 6, in some specific examples, the electronic device 600 may further include a communication module 607. The communication module 607 allows the electronic device 600 to exchange information/data with other devices through a computer network such as the Internet and/or various telecommunications networks, and may include, but is not limited to, a modem, a network card, an infrared communication device, a wireless communication transceiver and/or a chipset, such as a Bluetooth device, a WiFi device, a WiMax device, a cellular communication device, and/or the like.

In some possible examples, the signal processing module may carry out weighting processing on the second signal to output a normalized fourth signal, and adjust weighting parameters according to the third signal; and carry out subtraction operation on the first signal and the second signal, so as to output the third signal. The signal processing module may further execute a physiological signal determination algorithm, so as to output a physiological signal on the basis of the third signal, where the physiological signal may include a heart rate, blood oxygen, etc.

**In** some possible examples, the signal processing module may use a neural network model to process the first signal and the second signal, so as to output the third signal through the neural network model, where the third signal is a physiological signal, and the physiological signal may include a heart rate, blood oxygen, etc.

**In** some realizations, the determined physiological signal may be displayed through the display screen 606. **In** some realizations, the determined physiological signal may be sent to the other devices through the communication module 607. Specifically, if the electronic device 600 is a wearable device such as a bracelet or a watch, the determined physiological signal may be displayed in the display screen 606 of the electronic device 600, and may be sent to a smartphone which is paired with the electronic device 600 through the communication module 607, so as to be displayed or stored in the smartphone.

The example of the present application further provides a physiological signal sensing method, and the physiological signal sensing method may be applied to the above physiological signal sensing apparatus 100 and physiological signal sensing apparatus 200, and electronic device 600.

FIG. 7 is a flow diagram of a physiological signal sensing method which is provided by the example of the present application, and as shown in FIG. 7, the physiological signal sensing method may include the following steps.

Step S701, emitting first light at biological tissue.

Step S702, detecting second light obtained through reflecting the first light via the biological tissue and generating a corresponding first signal.

In the example of the present application, in an electronic device 100, in the case that a physiological signal sensing function is enabled, a processor 604 may control one or more light emitters 601 to emit light corresponding to the physiological signal sensing function, and control one or more photoelectric sensors 602 to detect light obtained through reflecting the light via biological tissue and generate a corresponding first signal. Specifically, during heart rate detection, the processor 604 controls the one or more light emitters 601 to emit light with a specific wavelength (usually a green light), and the light illuminates the surface of the skin. Due to the fact that blood has specific absorption characteristics for light with specific wavelengths, a light signal is absorbed by hemoglobin in the blood while penetrating through the skin and the tissue. The light passing through the tissue is partially absorbed and partially reflected or transmitted. Due to the fact that heartbeats cause periodic changes of a blood volume, these changes will cause intensity changes of the reflected or transmitted light. The reflected or transmitted light is received by the one or more photoelectric sensors 602, and received light signals are converted into electric signals. These electric signals include an alternating-current (AC) component and a direct-current (DC) component, where the AC component corresponds to blood volume changes which are synchronized with the heartbeats, while the DC component reflects optical characteristics of the tissue, as well as blood volumes of veins and arteries. Heart rate information may be extracted from the first signal through time-domain analysis or frequency-domain analysis. The time-domain analysis usually involves calculating a number of wave peaks within a certain period of time, while the frequency-domain analysis may involve identifying frequency components of a heart rate through fast Fourier transform (FFT).

Step S703, detecting changes of a capacitor which is in contact with the biological tissue and generating a corresponding second signal.

In the example of the present application, in the electronic device 600, the processor 604 may control the capacitive sensor 603 to detect changes of a capacitor which is in contact with the biological tissue and generate a corresponding second signal.

In a specific realization, the second signal may be generated through detecting self-capacitance of at least one electrode, and the detection may be realized through one electrode in the example. The second signal may also be generated through detecting mutual capacitance of at least one group of electrodes, each group of electrodes includes two electrodes, and position changes of the plurality of electrodes relative to the biological tissue can be better embodied in the example. The second signal may also be generated through detecting the above self-capacitance and mutual capacitance, such as detecting the self-capacitance of the first electrode to generate a first sub-signal, and detecting the mutual capacitance of the second electrode and the third electrode to generate a second sub-signal, where the second signal may include the first sub-signal and the second sub-signal.

In some possible examples, the processor 604 may control the one or more light emitters 601 to emit light in the first period, control the one or more photoelectric sensors 602 to detect reflected light and generate a first signal, and control the capacitive sensor 603 to detect changes of a capacitor and generate a second signal in the second period, where the second period is different from the first period.

If the one or more photoelectric sensors 602 are multiplexed as the capacitive sensor 603, the processor 604 may control the one or more photoelectric sensors 602 to detect light obtained through reflecting the light emitted by the one or more light emitters 601 via biological tissue and generate a corresponding first signal in the first period, and detect changes of a capacitor which is in contact with the biological tissue and generate a corresponding second signal in the second period. Specifically, the processor 604 may, in the first period, control the one or more light emitters 601 to emit first light, and control the switch module 6031 to enable the photoelectric sensors 602 to work in a photoelectric detection mode, so as to generate a first signal; and in the second period, control the one or more light emitters 601 to stop emitting the first light, and control the switch module 6031 to enable the photoelectric sensors 602 which are multiplexed as the capacitive sensor 603 to work in a contact detection mode, so as to generate a second signal. Specifically, in a realization, a specific photoelectric sensor 602 may be controlled to work in the contact detection mode, and the photoelectric sensor 602 is used as an electrode, and self-capacitance of the electrode is detected to generate the second signal. In another realization, two specific photoelectric sensors 602 may be controlled to work in the contact detection mode, the two photoelectric sensors 602 are used as electrodes, and mutual capacitance of the two electrodes is detected to generate the second signal.

In some specific examples, the motion artifact elimination is not realized through continuously generating the second signal, but through generating the second signal when a user with the electronic device 600 is in motion, otherwise, the motion artifact elimination is not carried out. When the user with the electronic device 600 is not in motion, the motion artifact elimination for the first signal may degrade the first signal due to the absence of motion artifacts. In the example of the present application, any suitable technology may be used for determining whether the user is in motion or not, such as using a motion sensor.

Step S704, carrying out motion artifact elimination on the first signal according to the second signal, so as to obtain a third signal.

In some possible examples, in the electronic device 600, the signal processing module may carry out weighting processing on the second signal to output a normalized fourth signal, and adjust weighting parameters according to the third signal; and carry out subtraction operation on the first signal and the second signal, so as to output the third signal. Specifically, a self-adaptive filtering unit is used for carrying out weighting processing on the second signal to output a normalized fourth signal, and weighting parameters of the self-adaptive filtering unit are adjusted according to the third signal; and an elimination unit is used for carrying out subtraction operation on the first signal and the second signal, so as to output the third signal. The signal processing module may further execute a physiological signal determination algorithm, so as to output a physiological signal on the basis of the third signal, where the physiological signal may include a heart rate, blood oxygen, etc.

In some possible examples, in the electronic device 600, the signal processing module may use a neural network model to process the first signal and the second signal, so as to output the third signal through the neural network model, where the third signal is a physiological signal.

In some realizations, the determined physiological signal may be displayed through the display screen 606. In some realizations, the determined physiological signal may be sent to the other devices through the communication module 607. Specifically, if the electronic device 600 is a wearable device such as a bracelet or a watch, the determined physiological signal may be displayed in the display screen 606 of the electronic device 600, and may be sent to a smartphone which is paired with the electronic device 600 through the communication module 607, so as to be displayed or stored in the smartphone.

The example of the present application provides an electronic device, and the electronic device includes: one or more light emitters which are used for emitting first light; one or more photoelectric sensors which are used for detecting second light obtained through reflecting the first light via biological tissue and generating a corresponding first signal; a capacitive sensor which is used for detecting changes of a capacitor which is in contact with the biological tissue and generating a corresponding second signal; a processor; and a memory storing a program, where the program includes instructions, and the processor is enabled to execute the above physiological signal sensing method when the instructions are executed by the processor. In some possible examples, the one or more photoelectric sensors are multiplexed as the capacitive sensor.

In the related art, a motion sensor carries out a solution of motion artifact elimination, the motion sensor is located in an electronic device, while a PPG sensor is in contact with biological tissue, and spatial correlation between the motion sensor and the PPG sensor is not high, so that a low elimination effect is caused. According to the physiological signal sensing method and apparatus, and the electronic device, which are provided by the example of the present application, the photoelectric sensors detect the second light obtained through reflecting the first light which is emitted by the light emitters via the biological tissue and generate the corresponding first signal, the capacitive sensor detects the changes of the capacitor which is in contact with the biological tissue and generates the corresponding second signal, both the photoelectric sensors and the capacitive sensor are in contact with the biological tissue, so that spatial correlation between the first signal and the second signal which are generated by the photoelectric sensors and the capacitive sensor is higher, and then a better motion artifact elimination effect is achieved. Further, in the example that the photoelectric sensors are multiplexed as the capacitive sensor, the photoelectric sensors are enabled to sense light information and detect electric information on an original basis, so that while respective advantages of capacitance detection and optical detection are combined, design complexity of a circuit does not need to be increased.

The above is merely the preferred examples of the present application, and does not limit the present application in any form, although the present application is disclosed above through the preferred examples, the present application is not limited thereto, any person skilled in the art may make slight changes or modifications to the technical contents disclosed above into equivalent examples with equivalent variations, without departing from the scope of the technical solutions of the present application, and any brief changes as well as equivalent variations and modifications made to the above examples according to the technical essence of the present application without departing from the contents of the technical solutions of the present application still fall within the scope of the technical solutions of the present application.

## Claims

1. A physiological signal sensing apparatus, comprising:
one or more light emitters, used for emitting first light;
one or more photoelectric sensors, used for detecting second light obtained through reflecting the first light via biological tissue and generating a corresponding first signal;
a capacitive sensor, used for detecting changes of a capacitor which is in contact with the biological tissue and generating a corresponding second signal; and
a signal processing module, used for carrying out motion artifact elimination on the first signal according to the second signal, so as to obtain a third signal.

2. The physiological signal sensing apparatus according to claim 1, wherein the one or more photoelectric sensors are multiplexed as the capacitive sensor.

3. The physiological signal sensing apparatus according to claim 2, wherein the second signal is generated through detecting self-capacitance of at least one of the photoelectric sensors, and/or mutual capacitance of at least one group of the photoelectric sensors, and each group of the photoelectric sensors comprises two photoelectric sensors.

4. The physiological signal sensing apparatus according to claim 2, wherein further comprising: an analog front-end, the analog front-end comprises:
a switch module, used for controlling a working mode of the photoelectric sensors, so as to enable the photoelectric sensors to generate the first signal or the second signal;
a driving module, used for driving the photoelectric sensors to generate the second signal; and
an analog signal processing module, used for processing the first signal and the second signal.

5. The physiological signal sensing apparatus according to any one of claims 1 to 4, wherein
the one or more light emitters are configured to emit the first light in a first period, and the one or more photoelectric sensors are configured to generate the first signal in the first period; and
the capacitive sensor is configured to generate the second signal in a second period, wherein the second period is different from the first period.

6. The physiological signal sensing apparatus according to any one of claims 1 to 4, wherein the signal processing module comprises:
a self-adaptive filtering unit which is used for carrying out weighting processing on the second signal to output a normalized fourth signal, and adjusting weighting parameters according to the third signal; and
an elimination unit which is used for carrying out subtraction operation on the first signal and the second signal, so as to output the third signal.

7. The physiological signal sensing apparatus according to any one of claims 1 to 4, wherein the signal processing module is configured to use a neural network model to process the first signal and the second signal, so as to output the third signal through the neural network model, wherein the third signal is a physiological signal.

8. A physiological signal sensing method, comprising:
emitting first light at biological tissue;
detecting second light obtained through reflecting the first light via the biological tissue and generating a corresponding first signal;
detecting changes of a capacitor which is in contact with the biological tissue and generating a corresponding second signal; and
carrying out motion artifact elimination on the first signal according to the second signal, so as to obtain a third signal.

9. The method according to claim 8, wherein the first light is emitted, the second light is detected, and the first signal is generated in the first period, and the changes of the capacitor are detected, and the second signal is generated in the second period, wherein the second period is different from the first period.

10. The method according to claim 9, wherein the second light is detected and the first signal is generated through one or more photoelectric sensors in the first period, and the one or more photoelectric sensors are used as electrodes for detecting the changes of the capacitor and generating the second signal in the second period.

11. The method according to claim 8, wherein the carrying out motion artifact elimination on the first signal according to the second signal, so as to obtain a third signal comprises:
carrying out weighting processing on the second signal to output a normalized fourth signal, and adjusting weighting parameters according to the third signal; and
carrying out subtraction operation on the first signal and the second signal, so as to output the third signal.

12. The method according to claim 8, wherein the carrying out motion artifact elimination on the first signal according to the second signal, so as to obtain a third signal comprises:
using a neural network model to process the first signal and the second signal, so as to output the third signal through the neural network model, wherein the third signal is a physiological signal.

13. An electronic device, comprising the physiological signal sensing apparatus according to any one of claims 1 to 8.

14. An electronic device, comprising:
one or more light emitters which are used for emitting first light;
one or more photoelectric sensors which are used for detecting second light obtained through reflecting the first light via biological tissue and generating a corresponding first signal;
a capacitive sensor which is used for detecting changes of a capacitor which is in contact with the biological tissue and generating a corresponding second signal; and
a processor; and
a memory storing a program,
wherein the program comprises instructions, and the processor is enabled to execute the method according to any one of claims 8 to 12 when the instructions are executed by the processor.

15. The electronic device according to claim 14, wherein the one or more photoelectric sensors are multiplexed as the capacitive sensor.
